(19) Europäisches Patentamt — European Patent Office — Office européen des brevets

(11) **EP 3 290 002 B1**

(12) **EUROPÄISCHE PATENTSCHRIFT**

(45) Veröffentlichungstag und Bekanntmachung des Hinweises auf die Patenterteilung:
**09.10.2019 Patentblatt 2019/41**

(21) Anmeldenummer: **17001574.7**

(22) Anmeldetag: **20.08.2012**

(51) Int Cl.:
*A61F 2/00* (2006.01)  *A61F 5/00* (2006.01)
*A61M 3/02* (2006.01)  *A61M 1/00* (2006.01)

(54) **VORRICHTUNG ZUR TRANS-ANALEN DRAINAGE VON STUHL AUS DEM REKTUM EINES PATIENTEN UND/ODER ZUR TRANS-ANALEN APPLIKATION VON EINLAUFFLÜSSIGKEIT DURCH EIN KATHETERARTIGES ELEMENT**

DEVICE FOR THE TRANS-ANAL DRAINAGE OF STOOL FROM THE RECTUM OF A PATIENT AND/OR FOR THE TRANS-ANAL APPLICATION OF INFLOWING LIQUID THROUGH A CATHETER-LIKE ELEMENT

DISPOSITIF DE DRAINAGE TRANSANAL DES SELLES HORS DU RECTUM D'UN PATIENT ET/OU D'APPLICATION TRANSANALE D'UN LIQUIDE DE LAVEMENT AU MOYEN D'UN ÉLÉMENT DU TYPE CATHÉTER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.08.2011 DE 102011111225**
**21.11.2011 DE 102011118943**
**16.12.2011 DE 102011121202**
**25.04.2012 DE 102012008361**
**03.05.2012 DE 102012008663**

(43) Veröffentlichungstag der Anmeldung:
**07.03.2018 Patentblatt 2018/10**

(62) Dokumentnummer(n) der früheren Anmeldung(en) nach Art. 76 EPÜ:
**12761540.9 / 2 744 444**

(73) Patentinhaber: **Advanced Medical Balloons GmbH**
**68753 Waghäusel (DE)**

(72) Erfinder: **Göbel, Fred**
**69259 Wilhelmsfeld (DE)**

(74) Vertreter: **Küchler, Stefan**
**Patentanwalt**
**Färberstrasse 20**
**90402 Nürnberg (DE)**

(56) Entgegenhaltungen:
**WO-A1-2008/103788**

**Beschreibung**

Die Erfindung richtet sich auf eine Vorrichtung zur trans-analen Drainage von Stuhl aus dem Rektum eines Patienten durch ein katheterartiges Element mit einem rohrförmigen Schaftkörper und einem aufblasbaren Ballonelement, und/oder zur trans-analen Applikation von Einlaufflüssigkeit durch ein katheterartiges Element mit einem rohrförmigen Schaftkörper und einem aufblasbaren Ballonelement, der aus einem dünnwandigen Weichfolienmaterial von begrenzter elastischer Verformbarkeit unter Präformierung mit einer taillierten- oder hantelförmigen oder sanduhrförmigen Gestalt hergestellt ist, mit zwei endständigen Ballonabschnitten von größerem Schlauchradius sowie einem dazwischen angeordneten, mittigen, demgegenüber verjüngten Ballonabschnitt von verringertem Schlauchradius, welcher trans-anal platziert wird, derart, dass der distal anschließende, radial erweiterte Ballonabschnitt intra-rektal platziert ist und der proximal anschließende, radial erweiterte Ballonabschnitt extra-korporal.

Vorrichtungen zur vorübergehenden trans-analen Ableitung (Drainage) von Stuhl sind in verschiedenster Ausführungsform seit längerem bekannt (siehe z.B. WO2008103788 A).

Die Patentschrift DE 10 2004 033 425 B4 beschreibt ein Verschlußsystem zur Versorgung rektaler bzw. analer Inkontinenz mit einer besonderen Ausführungsform für die Tamponade blutender Hämorrhoiden, wobei ein tailliertes Ballonelement dargestellt ist, welches endständig über einen intra-rektalen und prä-analen Abschnitt verfügt. Die Ballonhülle ist dabei derart auf dem ballontragenden Schaftkörper platziert, dass sich das intra-rektale Ballonsegment bei Befüllung zum Rektumboden hin bewegt und dort auf die blutenden venösen Gefäße einen tamponierenden Druck ausübt. Entsprechend bewegt sich das prä-anale Ballonsegment zur äußeren Analöffnung hin. So stellt sich eine von beiden Seiten axial auf den Anus gerichtete Tamponadewirkung ein. Zusätzlich zur axialen Blutungstamponade dehnt sich der mittlere taillierte Abschnitt des Ballons radial zur Wandung des Analkanals hin aus. Er ist bevorzugt mit einem Durchmesser ausgeformt, der den Durchmesser des geöffneten Analkanals überschreitet. Die Spitze des Schaftkörpers ragt bei der beschrienen Vorrichtung zur Akutversorgung einer venösen ano-rektalen Blutung im Zustand der Anwendung frei und ungeschützt in den Darmraum, und stellt bei einer, über eine Intervention hinaus gehende Liegedauer, ein erhebliches Verletzungsrisiko dar.

Unter dem selben Nachteil leidet die Anordnung gemäß der WO2007/118621 A1. Diese beschreibt ebenfalls ein hantel- bzw. sanduhrförmiges Ballon-Verschlußsystem. Ihm liegt die Aufgabe zu Grunde, für kurze intermittierende Zeiträume von wenigen Stunden bei chronisch ano-rektal inkontinenten Patienten eine Dichtung gegen unwillkürlich austretenden Stuhl zu gewährleisten. Bei dieser Vorrichtung zur Versorgung steht ebenfalls die von beiden Seiten axial zum Anus hin gerichtete Rollbewegung der endständigen Ballonsegmente im Vordergrund. Auch hier ragt jedoch die Spitze des den Ballon tragenden Schaftkörpers im trans-anal platzierten Zustand frei und potentiell traumatisierend in den Darmraum, was eine gefahrlose längerfristige Anwendung des schaftintegrierenden Verschlußkörpers nicht gestattet.

Aus diesen Nachteilen des Standes der Technik resultiert das die Erfindung initiierende Problem, bei einer gattungsgemäßen Anordnung Sorge dafür zu tragen, dass bei längerfristiger Platzierung des Schaftkörpers im Anus eine permanente Aufweitung (Dilatation) der analen Strukturen, wie sie bei einem starren, nicht kollabierbaren Rohrkörper zu erwarten wäre, vermieden werden kann.

Die Lösung dieses Problems gelingt bei einer gattungsgemäßen Vorrichtung dadurch, dass die beiden Ballonenden sich verjüngen und in einem axialen Abstand voneinander auf der Mantelfläche des Schaftkörpers fixiert sind, wobei der intra-rektale Ballonabschnitt in kräftefreiem Zustand den distalen Bereich des Schaftkörpers vollständig umgibt und derart bemessen ist, dass bei einer darmwärts gerichteten axialen Auslenkung des Schaftkörpers infolge der damit verbundenen Verformung der das Schaftende umgebenden Ballonhülle eine entgegengesetzt wirkende Kraft erzeugt wird, welche das distale Ende des Schaftkörpers in seiner Bewegung nach distal dämpft und/oder begrenzt, und wobei der Schaftkörper derartige elastische Verformbarkeits- und Wiederaufrichtungseigenschaften aufweist, dass er bei normalem anlastenden analen Schließmuskeltonus in einen radial eingefalteten Zustand übergeht oder auf ein im Durchmesser kleineres Maß kollabiert, bei Nachlassen des Schließmuskeltonus sich jedoch spontan wieder auf sein ursprüngliches Durchmessermaß aufrichtet.

Die radiale Faltbarkeit soll sicherstellen, dass bei längerfristiger Platzierung des Schaftkörpers im Anus eine permanente Aufweitung (Dilatation) der analen Strukturen, wie sie bei einem starren, nicht kollabierbaren Rohrkörper zu erwarten wäre, vermieden werden kann.

Die Erfindung ist definiert durch Anspruch 1.

Neben einer gegebenenfalls atraumatisch wirkenden Stauchung bzw. Knickung über die Schaftlängsachse ist der Schaft für eine gewebeverträgliche radiale Einfaltung des Schaftkörpers bei geringer wirkender Kraft ausgelegt. So soll sich der Schaftkörper bereits unter normalem anlastenden analen Schließmuskeltonus weitgehend radial einfalten bzw. auf ein im Durchmesser kleineres Maß kollabieren. Lässt der Schließmuskeltonus nach, soll sich der eingefaltete Schaftkörper wieder auf sein ursprüngliches Durchmessermaß aufrichten bzw. sich in den jeweiligen freigegebenen Analkanal hinein spontan öffnen.

Um eine möglichst atraumatische Platzierung des intra-korporalen Drainagesegments zu gewährleisten, wird der im Rektum und Anus liegende Anteil des ableitenden Schlauches vorzugsweise aus einem weichen, bei geringer von außen einwirkender Kraft faltbaren bzw. kollabierbaren Material ausgeführt. Der Analkanal wird also durch den ableitenden Schlauch nicht dauerhaft geweitet, was einer Schädigung der analen Strukturen auch bei langer Anwendungsdauer weitgehend vorbeugt. Die technische Ausführung solcher im Körper bis zu mehreren Wochen verbleibender Drainagen ist in der Regel aufwendig und schließt die sogenannte "indwelling drainage" für kürzere Anwendungsperioden aus Kostengründen daher weitgehend aus.

Zum Schutz vor bei der Faltung bzw. Stauchung des Schaftrohres entstehender, scharfkantiger und potentiell traumatisch auf anliegende Gewebe wirkender Knickstellen, beschreibt die vorliegende Erfindung eine weich verformbare, auf der Oberfläche des Schaftrohres vorzugsweise in fester Verbindung aufgebrachten Ummantelung, die das knick- bzw. faltbare Drainagerohr in seiner gesamten Länge umschließt oder zumindest den Balloninnenraum (Distanz zwischen den Fixierungspunkten des Ballons auf dem Schaftkörper) umfasst. Neben seiner protektiven Wirkung kann die Ummantelung auch eine elastische Eigenwirkung aufweisen, welche die elastischen Verformbarkeits- und Wiederaufrichtungseigenschaften des Schaftkörpers für die jeweils spezifischen Ausführungsformen der Vorrichtung vorteilhaft modifizieren kann.

Für die Ableitung dünnflüssiger Stühle sind in der Regel Schaftrohrinnendurchmesser von ca. 12 bis 20 mm ausreichend, bevorzugt werden Durchmesser von15 bis 18 mm.

Das Schaftrohr besteht bevorzugt aus elastisch wirkenden Polyurethanen (PUR) der Shore-Härten 60A bis 95A und weist bevorzugt Wandstärken von ca. 0,2 mm (bei 12 mm Innendurchmesser) bis zu ca. 0,5 mm (bei 22 mm Innendurchmesser) auf.

Bei der Wandstärkenbemaßung und der Wahl der Materialhärte (Shore-Härte) des Schaftrohres im angegebenen Innendurchmesserbereich von 12 bis 22 mm ist ferner zu beachten, dass der den Ballon tragende Schaftrohranteil ein ausreichend großes Selbstaufrichtungsvermögen besitzt, um dem auf der Schaftaußenseite anliegenden Fülldruck des Ballons entgegenzuwirken, und so einen lumeneinengenden oderverschließenden Kollaps des Drainagelumens zu vermeiden. Bei Fülldrucken bis ca. 45 mbar soll das drainierende Lumen des Schaftrohres, bei freier Entfaltung des Ballons außerhalb des Körpers, im Bereich seiner größten Verengung bevorzugt auf nicht mehr als 20% seines frei entfalteten Ausgangsmaßes reduziert sein, bei 25 mbar auf nicht mehr als 50%.

Zur weiteren Steigerung der lumen-erhaltenden, elastischen Eigenspannung des Schaftrohres kann dieses auch in einzelnen Abschnitten, wie vorzugsweise dem trans-analen Segment, durch eine Vergrößerung der Wandstärke, Erhöhung der Materialhärte oder einen segmentalen Wechsel der Materialart anpassend modifiziert werden.

Neben Polyurethanen sind als Schaftmaterialien ebenfalls denkbar: Silikone, SEBS, Kautschuk, PUR/PVC-Mischungen, oder auch PVC denkbar.

Die schützende Ummantelung des Schaftrohres kann als einfaches, durchgängig zylindrisches Element ausgeformt und aufgebracht werden, in weiteren Ausführungen der Vorrichtung jedoch auch als hantel- oder sanduhrförmiges Element ausgebildet sein bzw. an seinem distalen und proximalen Ende diskus- oder sphärische Erweiterungen aufweisen. Der ummantelnde Körper kann neben einer Ausführung als durchgängiges Element alternativ aus mehreren nicht verbundenen bzw. von einander beabstandeten Einzelsegmenten bestehen.

Die Ummantelung wird vorzugsweise aus Schaummaterial mit elastisch verformbaren, sich selbst-aufrichtenden Eigenschaften, wie diese beispielsweise von Polyurethanschäumen bekannt sind, ausgeführt. Die selbstaufrichtende Wirkung des Schaummantels kann unterstützend zur selbständigen Öffnung und gegebenenfalls auch axialen Entwindung eines gefalteten bzw. verformten Rohrlumens beitragen. Die Wandungsstärke des drainierenden Schaftrohres, welches dann durchgängig und fest mit dem umgebenden Schaummantel verbunden ist, kann bei einer Ausführung als Materialverbund von Schaft und Mantel optional reduziert werden. Hierdurch lassen sich die elastischen Eigenschaften des Rohrkörpers gegebenenfalls weiter verbessern und die Gewebefreundlichkeit des Drainagerohres im gefalteten bzw. verformten Zustand insgesamt optimieren. Durch eine abgestimmte Kombination der Elastizität des Rohrkörpers und der sich bei dessen Faltung bzw. Knickung einstellenden Rückstellkraft mit der elastischen Rückstellwirkung des aufsitzenden Schaummantels, lässt sich eine optimal niedrige lumen-aufrichtende Gesamtelastizität des Materialverbundes, bei gleichzeitiger bestmöglicher Protektion der anliegenden Gewebe erreichen.

Neben der Ausführung als elastisch, visko-elastisch oder auch nicht elastisch wirkender Schaumkörper, kann die Ummantelung ebenfalls als ein elastisch oder nicht elastisch wirkender, dem Schaftrohr wahlweise fest oder auch nicht fest aufsitzender Gelkörper, faser- oder watteartiger Körper, oder auch besonders weicher Vollkörper niedriger Shore-Härte, aus Materialien wie z.B. Silikon, Polyurethan oder SEBS ausgeführt werden. Die vorzugsweisen Shore-Härten derartiger Körper reichen von ca. 10A bis 70A, bevorzugt jedoch von 20A bis 50A.

Als besonders vorteilhaft bei schaumbasierten Ummantelungen gelten Shore-Härten des Bereichs von 30A bis 90 A, bevorzugt jedoch 40 bis 70A. Die entsprechenden Wandungsstärken der Schaumummantelung reichen von bevorzugt ca. 1,5 mm bis 4,0 mm, besonders vorteilhaft von 2,0 bis 3,0 mm.

Die elastischen Selbstaufrichtungseigenschaften der Ummantelung des Schaftrohres können ferner eingesetzt werden, um dem so ausgerüsteten Verbund-Schaftkörper die nötige Widerstandsfähigkeit gegen den, dem Schaft von außen anlastenden Ballon-Fülldruck zu geben, welcher bei entsprechender Größe zum lumeneinengenden partiellen Kollaps des drainierenden Rohrlumens führen kann. Bei Fülldrucken bis ca. 45 mbar soll das drainierende Lumen des Schaftrohres, bei freier Entfaltung des Ballons außerhalb des Körpers, im Bereich seiner größten Verengung bevorzugt auf nicht mehr als 20% seines frei entfalteten Ausgangsmaßes reduziert sein, bei 25 mbar auf nicht mehr als 50%.

Ferner beschreibt die Erfindung besondere Anordnungen bzw. strukturelle Verbindungen des darmwärts gerichteten, distalen Endes der Ummantelung in Bezug zu den, den vorderen, frontalen Radius des intra-rektalen Ballonsegmentes bildenden Anteilen der Ballonhülle sowie zu dem vorderen, distalen Ende des den die Ummantelung tragenden Schaftrohres.

In der späteren Folge werden im Rahmen konkreter Ausführungsbeispiele das elastische Faltungs- und Aufrichtungsverhalten eines schaumarmierten Drainagerohres sowie eines Drainagerohres ohne Ummantelung bei axial und radial am Schaftkörper anlastender Kraft beschrieben.

Zur Optimierung der ankernden und dichtenden Funktion sowie der Gewebeverträglichkeit der beschriebenen Drainagevorrichtung schlägt die Erfindung die Verwendung besonders dünnwandiger Ballonfolien aus Materalen mit vorzugsweise geringer Volumendehnbarkeit vor. Die Ballonkörper werden bereits bei der Herstellung auf ihr Arbeitsmaß ausgeformt. Die Befüllung Ballonelementes erfolgt in situ vorzugsweise mit einem Volumen, welches kleiner ist als das Volumen des dem Schaft aufsitzenden Ballons bei dessen freier, nicht mit Druck beaufschlagter Entfaltung außerhalb des Körpers. Hierdurch wird eine Dehnung der Ballonhülle ausgeschlossen. Die im Ballon wirkende Kraft entspricht bei einer derartigen inkompletten Füllung lediglich der kumulierenden Wirkung des intra-rektalen Druckes, der anlastenden Kraft des trans-analen Sphinkters sowie der Kraft, welche von den Gesäßbacken auf den prä-analen Anteil der Ballonhülle ausgeübt wird. Im idealen Anwendungsfall werden sowohl die rektale Verankerung als auch die transanale Dichtung der Vorrichtung bereits durch den auf den Ballon einwirkenden physiologischen, intra-abdominellen Druck funktionell wirksam. Dies entspricht der geringst möglichen Kraftwirkung des Ballons auf die ihm anliegenden Gewebe.

Im Gegensatz zu den im Stand der Technik beschriebenen trans-analen Verschlussvorrichtungen bei chronisch stuhlinkontinenten Patienten, bei denen die analen Strukturen in vielen Fällen bereits degenerative Veränderung aufweisen und sich der Analkanal oft sehr uneinheitlich in Durchmesser und Länge darstellt, kommen Darmrohre der erfindungsgenmäßen Bauart überwiegend bei Patienten mit normaler ano-rektaler Anatomie zum Einsatz. Wegen der in der Regel normalen Anatomie kann auf eine von beiden Seiten axial auf den Anus hin gerichtete Gegenrollbewegung der endständigen Ballonanteile als Funktionsmerkmal zur spontanen Anpassung des Ballonkörpers an zum Teil sehr individuelle Anatomie hier verzichtet werden. Eine besondere Inversion der Ballonenden bei der Ballonmontage auf dem Schaftkörper ist daher nicht erforderlich. Das mittig liegende, verjüngte trans-anale Segment des Ballonkörpers sollte vorzugsweise eine Länge von 2 bis 5 cm, bevorzugt 2,5 bis 3,5 cm aufweisen, um den Analkanal in seiner gesamten, anatomisch normalen Längenausdehnung zwischen den endständigen Ballonanteilen aufnehmen zu können.

Im Gegensatz zum Stand der Technik gehen die im Rahmen der Erfindung beschriebenen trans-anal platzierten Drainagekatheter auch auf die atraumatische Sicherung der Schaftspitze innerhalb des intra-rektalen Ballonsegmentes bei einer körperwärts gerichteten axialen Auslenkung des Katheterschaftes ein. Perforationen der dem freien distalen Ende des Schaftkörpers exponierten Darmwand werden bei erfindungsgemäßer Ausführung der Vorrichtung weitestgehend vermieden.

Neben den erfindungsgemäß beschriebenen bevorzugten Techniken zur Vermeidung von Darmwandirritationen oder -perforationen durch entsprechende Ballon-Schaft Anordnungen, können die erfindungsgemäßen Merkmale jedoch auch mit den Bauprinzipien einer Gegenrollbewegung der endständigen Ballonerweiterungen bzw. einer von beiden Seiten axial dichtenden Ballonwirkung kombiniert werden. Die Erfindung stellt hierbei wiederum sicher, dass das freie distale Schaftende nicht in traumatisierender Weise in den Darm hinein- geschoben bzw. ausgelenkt wird.

Zur längerfristigen Versorgung stuhlinkontinenter Patienten über einen Zeitraum von bis zu mehreren Tagen und Wochen werden heute bevorzugt sogenannte Stuhldrainagen verwendet, eine Technologie, die als sogenannte "indwelling fecal drainage" bezeichnet wird. Die entsprechenden Drainagekatheter werden in der Regel mittels eines ballonartigen Ankerelementes im Patientenrektum platziert bzw. durch eine entsprechend flächige Auflage des Ankerballons auf dem Rektumboden vor dem unerwünschten Herausgleiten durch den Anus gesichert. Das die Vorrichtung rektal sichernde Ballonelement sitzt bei der Mehrzahl der im Markt eingeführten Drainagekatheter einem, den Stuhl trans-anal ableitenden Schlauchelement direkt auf. Ein proximal endständig am ableitenden Schlauch angebrachter, in der Regel auswechselbarer Sammelbehälter nimmt den Stuhl auf.

Neben Stuhldrainagen für die langfristige Platzierung finden zur Versorgung inkontinenter immobiler Patienten gegenwärtig sogenannte Fäkalkollektoren Verwendung. Im Gegensatz zu Stuhldrainagen bestehen diese im Wesentlichen aus einem, den Stuhl direkt aufnehmenden Folienbeutel. Fäkalkollektoren werden mittels am Beutel angebrachter Adhäsionsflächen direkt auf die peri-anale Haut aufgeklebt. Fäkalkollektoren gehen bei der Anwendung über mehrere Tage häufig mit einer Schädigung der peri-analen Epidermis einher, welche zum einen durch die verklebungsbedingte Irritation, und zum anderen durch die fortwährende mazerierende Einwirkung des Stuhls auf die exponierte Haut bedingt ist. Desweiteren können sich die Sammelbeutel lösen und entsprechende Verschmutzungen verursachen. Fäkalkollektoren kommen wegen der regelmäßig zu beobachtenden und zum Teil schweren peri-analen Irritationen in der Regel nur über kurze Zeiträume zur Anwendung.

Die konzeptionell einfachste Form der trans-analen Ableitung von Stuhl stellt das sogenannte Darmrohr dar. Es besteht aus einem relativ kurzen Rohrelement, welches am patientenabgewandten, also proximalen Ende mit einem trichterartigen Konnektor zum Anschluss eines ableitenden und sammelnden Auffangsystems versehen ist. Problematisch bei Darmrohren ist in erster Linie die sichere anale bzw. peri-anale Fixierung der Vorrichtung, die das Herausgleiten aus dem Anus sowie das unkontrollierte Eindringen des Rohres in das Rektum vermeidet. Das Risiko einer traumatischen Penetration von rektalen und höher gelegenen Darmwandanteilen durch die Spitze des in der Regel relativ starrwandig ausgeführten Darmrohrschaftes ist grundsätzlich gegeben und veranlasst viele Anwender von der Verwendung von Darmrohren ganz abzusehen.

Zur Gewährleistung einer besseren Fixierbarkeit werden konventionelle Darmrohre unter anderem mit einem Ballonelement ausgestattet, wobei dieses in der Regel am distalen, intra-rektalen platzierten Ende der Vorrichtung angebracht ist. Ferner sind Ausführungsformen mit zwei separaten Ballons im Gebrauch, wobei der intra-rektale Ballon durch einen vor dem Anus liegenden, prä-analen Ballon ergänzt wird. Die beiden, dem Schaftkörper jeweils separat aufsitzenden Ballons werden über eine gemeinsame Zuleitung befüllt, und haben im befüllten Zustand den Anus zwischen sich aufgenommen, wodurch die Lage des Darmrohrs weitgehend gesichert und eine Perforation weitgehend ausgeschlossen war. Dennoch kam es durch die unter Druck prall aufgedehnten Ballonelemente zu lokalen Reizungen der ihnen anliegenden Strukturen. Ebenfalls wurden im Bereich des Analkanals mechanisch bedingte Läsionen durch den, dem Gewebe dort direkt anliegenden harten Schaft des Rohres beobachtet. Für eine längerfristige atraumatische Platzierung im Rektum erwiesen sich Darmrohre mit Doppelballonausstattung in der damaligen Bauform daher als nicht geeignet.

Die vorliegende Erfindung beschreibt eine fortgeschrittene ano-rektale Darmrohrtechnik, die vorrangig für die nicht-irritierende, möglichst atraumatische Anwendung im Patienten über kurze Anwendungszeiträume von vorzugsweise drei bis vier Tagen und vorrangig auf die Versorgung von Patienten mit dünnflüssigen Stühlen ausgelegt ist.

Hierzu wird ein Katheterschaftkörper, welcher besondere axiale und radiale Stauchungs- bzw. Faltungseigenschaften aufweist, mit einem in besonderer Weise, distal auf dem Schaftkörper aufgebrachten, etwa hantel- oder sanduhrförmigen Ballonelement ausgestattet, welches bereits bei der Ballonherstellung auf das erforderliche Arbeitsmaß ausgeformt ist und somit zur Erreichung seiner, den Katheter trans-anal verankernden und dichtenden Funktionsform keine kraftintensive Dehnung seiner Ballonhülle erfordert.

Die angestrebte atraumatische Wirkung der erfindungsgemäßen Vorrichtung wird im Rahmen der Erfindung vorzugsweise durch eine Kombination der besonderen elastischen Verformungs- und Aufrichtungseigenschaften des den Ballon tragenden Schaftes und einer besonderen Ballonformgebung sowie Positionierung und Fixierung der Ballonkomponente auf dem tragenden Schaft ermöglicht.

Ferner hat es sich bewährt, dass bei weitergehender, darmwärts gerichteter, axialer Auslenkung des proximalen Schaftendes der den Ballon tragende Schaft in den Zustand einer einfachen oder mehrfachen axialen Stauchung übergeht.

Die Erfindung zeichnet sich dabei aus durch eine mehrschrittige Pufferung einer Schaft-Auslenkung.

durch einen Toleranzbereich mit einfachem, relativen Spiel zwischen Katheterschaft und Ballon, d.h., mit einer minimalen, rückstellenden Kraft $F_r \approx 0$, bei einer Auslenkung x gegenüber dem neutralen, kräftefreien Zustand um einen Wert bis zu einem ersten Grenzwert $G_1$:

$$x \leq G_1 \Rightarrow F_r = 0 \pm \varepsilon,$$

wobei $|\varepsilon| \leq 10$ g, vorzugsweise $|\varepsilon| \leq 5$ g, insbesondere $|\varepsilon| \leq 2$ g.

Ferner gibt es jenseits dieses Toleranzbereichs eine Rückstellkraft durch Verformung des intrarektalen Ballonabschnitts, d.h., mit einer rückstellenden Kraft $F_r$ der Ballonhülle, bei einer Auslenkung x gegenüber dem neutralen, kräftefreien Zustand um einen Wert zwischen einem ersten Grenzwert $G_1$ und einem zweiten Grenzwert $G_2 > G_1$:

$$G_1 \leq x \leq G_2 \Rightarrow F_r \approx c_H * (x - G_1),$$

wobei $c_H$ der Federkonstante der drucklos aufgeblähten Ballonhülle entspricht. Diese Federeigenschaft wird neben den Materialeigenschaften der Hülle auch von der präformierten Geometrie der Ballonhülle bestimmt.

Oberhalb eines zweiten Grenzwertes gibt es eine Rückstellkraft durch Verformung (z.B. axiale Knickung oder Stauchung) des Katheterschaftes, d.h., mit einer rückstellenden Kraft $F_r$ des Katheterschaftes, bei einer Auslenkung x gegenüber dem neutralen, kräftefreien Zustand um einen Wert oberhalb des zweiten Grenzwerts $G_2$:

$$G_2 \leq x => F_r = c_K * (x - G_2),$$

wobei $c_K$ der (kleinsten) Federkonstante des Katheterschafts entspricht. Bei verschiedenen Querschnittsgeometrien des Katheterschaftes, bspw. mit vordefinierten Schwächungen, ist hierfür derjenige Bereich anzusetzen mit der geringsten Federkonstante $c_K$, weil dieser Bereich als erstes nachgibt.

Um eine Perforation der Wandung des Rektums oder des sich an das Rektum anschließenden Sigmas durch den ballontragenden Katheterschaft auszuschließen, wird dieser mit der Fähigkeit ausgestattet, bei forcierten akzidentellen, darmwärts gerichteten Auslenkungen des Schaftkörpers, noch vor dem Erreichen potentiell schädigender Wirkungen der Schaftspitze auf die Darmwandung, über die Längsachse des Schaftes hinweg, in Form einer oder mehrerer axialer Knickungen bzw. Faltungen, in einen elastisch gestauchten Zustand überzugehen, und mit einsetzender Stauchung des Schaftes eine weitere darmwärts gerichtete Auslenkung der Schaftachse derart aufzunehmen, dass die auf die Schaftspitze bzw. den ihr eventuell anliegenden Darmwandanteilen wirkende Kraft weitgehend konstant gehalten werden kann. Bei Entlastung der Schaftachse von der von proximal auf sie einwirkenden Kraft geht der sich elastisch wirkende Schaftkörper spontan wieder in seinen lumenoffenen, durchgängig längs ausgerichteten Ausgangszustand über.

Neben dieser elastischen Stauchung des Schaftkörpers im Falle einer massiven, lang-streckigen Auslenkung des Schaftes, beschreibt die Erfindung spezifische Positionierungen des taillierten Ballonelementes auf dem den Ballon tragenden Schaftkörper, die kurz-streckige Auslenkungen des Schaftes innerhalb des trans-anal platzierten Ballons in besonderer Weise atraumatisch puffernd aufnehmen und das den Stuhl drainierende Schaftlumen dabei im Rahmen des so ermöglichten relativen axialen Spiels zwischen Schaftkörper und Ballon weitgehend geöffnet halten bzw. eine Knickung des Schaftkörpers durch moderate Schaftauslenkungen vermeiden.

Der trans-anal dichtend wirkende und den Katheterschaft dislokationssicher im Analkanal verankernde taillierte Ballon wird im Rahmen der Erfindung in besonderer Art und Weise auf dem den Ballon tragenden Schaftkörper fixiert. Die Schaftenden des Ballons werden dabei in besonderer Weise in Bezug zum distalen, freien Ende des Schaftkörpers gebracht, wodurch sich das distale Ende des Schaftkörpers in spezifischer Weise zum distalen Radius des intra-rektalen Ballonsegmentes positioniert.

Bei den verschiedenen erfindungsgemäß beschriebenen Ausführungen der relativen Anordnung von Ballon und Schaft steht jeweils die Vermeidung einer darmwärts gerichteten, unkontrollierten, potentiell perforierenden Auslenkung des distalen Schaftendes aus dem intra-rektalen Ballonanteil heraus im Vordergrund.

Die Erfindung beschreibt sowohl Ballon-Schaft Anordnungen mit zueinander hin versetzten Ballonschaftenden und einer daraus resultierenden axialen Gegenrollbewegung der endständigen Ballonerweiterungen, als auch Ballon-Schaft Anordnungen mit geringem bzw. ohne jeglichen Versatz der Ballonschaftenden, in letzterem Fall in einer Positionierung, wie sie sich die Positionierung des Ballonenden spontan bei freier, spannungsloser Entfaltung des Ballons auf dem Schaft darstellen würde.

Es werden bei der erfindungsgemäßen Ballon-Schaftanordnung verschiedene axial gerichtete Auslenkungsgrade des Schaftkörpers innerhalb des Ballonelementes beschrieben. Berücksichtigt werden sowohl geringgradige freie relative Bewegungen zwischen Schaft und Ballon, als auch weitere Auslenkungen des Schaftkörpers, die über das freien relative Spiel der beiden Komponenten hinausgehen und in einer elastischen Verformung der distalen Ballonhüllenanteile aufgefangen werden. Kommt es, über diesen Auslenkungspunkt hinaus, zu noch länger-streckigen darmwärts gerichteten Bewegungen des Schaftes, oder eventuell zu einer vollständigen Dislokation des gesamten ballontragenden Anteils der Drainage aus der trans-analen Platzierung heraus in den Darm hinein, wird durch die besondere Gestaltung des Schaftkörpers bzw. dessen spezifischen elastischen Verformbarkeits- und Wiederaufrichtungseigenschaften, einer Perforation von Darmanteilen vorgebeugt, indem der den Ballon tragende Schaftkörper in den Zustand einer axialen Knickung oder Stauchung übergeht. Kommt es zum Beispiel im Rahmen einer Katheterdislokation zu einer forcierten Kollision der vorderen Drainageanteile mit Anteilen der Darmwandung, staucht sich der sich elastisch faltende Schaftkörper über seine Längsachse hinweg in Form einer oder mehreren Knickungen ein, und nimmt so die jeweilige Auslenkung des Schaftes weitgehend kraft-neutralisierend auf. Bei bevorzugter Verwendung von Polyurethanen als Basismaterial für den Schaftkörper gestaltet sich die Stauchung bzw. Knickung derart, dass der Schaft nach Überwindung einer initialen, von axial her auf den Schaftkörper wirkenden Kraft unmittelbar scharf knickt und der weitere Verlauf der streckenverkürzenden Stauchung dann bei deutlich geringerer axial auf die Schaftspitze einwirkender Kraft erfolgen kann.

Bei Entlastung der jeweilig auf den Schaft einwirkenden Kraft, richtet sich der Schaftkörper sukzessive wieder spontan in seine lumenoffene ursprüngliche Längsform auf.

In ähnlicher Weise verhält sich der elastische Schaftkörper, wenn es im Rahmen einer Massenbewegung oder Lageänderung des Patientenkörpers zu einer einfachen axialen Knickung des Schaftes im Sinne einer Abwinkelung des trans-analen Segmentes zum intra-rektalen Segment des Schaftkörpers kommt. Der elastische Schaftkörper knickt hierbei ebenfalls bevorzugt bereits bei geringer wirkender Kraft um einen entsprechenden Winkelbetrag ein, und richtet sich bei Rückkehr in die Körper-Normallage spontan wieder in seiner Längsachse aus.

Fig. 1 zeigt eine Ausführungsform der Vorrichtung, bei der das distale, darmwärts gerichtete Ende des Schaftrohres nahezu bündig mit dem distalen Schaftende der Ballons verbunden ist, und bei freier, nicht mit Druck beaufschlagter Entfaltung der Ballonhülle, nahezu bündig mit dem frontalen Radius des intra-rektalen Ballonsegmentes abschließt.

Fig. 2 zeigt den kombinierten dämpfend schützenden und elastisch lumenaufrichtenden Effekt einer Ummantelung des Schaftrohres bei einer sich einstellenden radialen Verformung bzw. Einfaltung des Schaftkörpers im Querschnitt durch den Drainagekörper.

Die Fig. 3a bis 3c zeigen schematisch und beispielhaft eine taillierte, im mittigen Abschnitt sanduhr- bzw. hantelförmig verjüngte Ballonhülle, bei räumlich freier, nicht mit Druck beaufschlagter Entfaltung.

Fig. 4 zeigt die in Fig. 1 beschriebene Ausführungsvariante bei trans-analer Platzierung der Vorrichtung, im Zustand einer darmwärts gerichteten, axialen Auslenkung des Schaftrohres innerhalb des dem Schaftrohr aufsitzenden Ballonkörpers um den Betrag A, welche zu einer entsprechenden, die Auslenkung des Schaftes zum Darm hin begrenzenden, elastischen Verformung der vorderen Hüllenanteile des intra-rektalen Ballonsegmentes um den Betrag A führt.

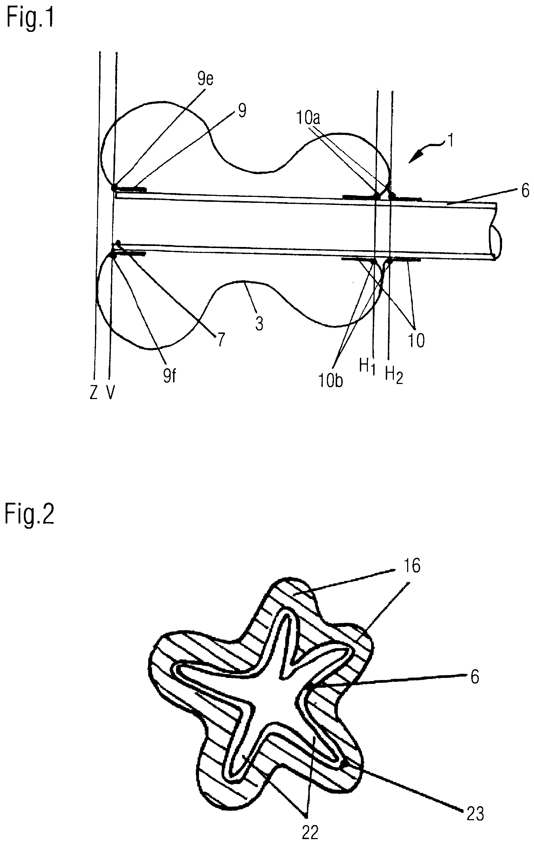

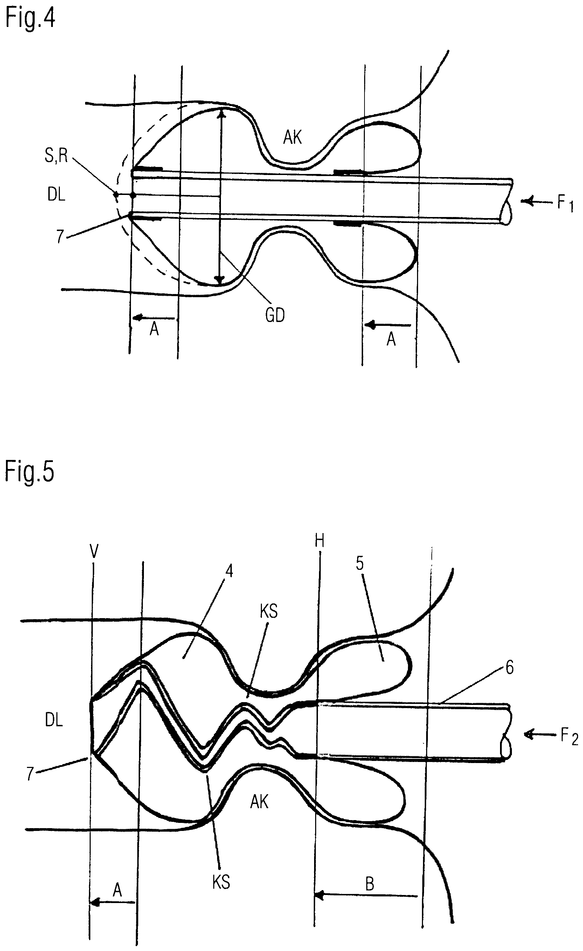

Fig. 5 zeigt die in Fig. 1 bzw. Fig. 4 beschriebene Ausführungsvariante bei transanaler Platzierung der Vorrichtung, im Zustand einer darmwärts gerichteten, axialen Auslenkung des Schaftrohres innerhalb des dem Schaftrohr aufsitzenden, trans-anal platzierten Ballonkörpers um den Betrag B, welche nicht durch eine, die Auslenkung des Schaftes begrenzende, elastische Verformung der Hüllenanteile des intrarektalen Ballonsegmentes aufgefangen werden kann, sondern stattdessen in eine einfache oder mehrfache Faltung bzw. Knickung des Schaftrohres über seine Längsachse übergeht, wobei die vorderen Hüllenanteile des intra-rektalen Ballonsegmentes wiederum lediglich um den Betrag A darmwärts ausgelenkt werden.

Fig. 6 zeigt eine weitere Ausführungsvariante der Vorrichtung, bei der die Schaftenden des Ballonkörpers, bei freier, nicht mit Druck beaufschlagter Entfaltung des Ballons, beidseits um einen Betrag C invertiert, und in dieser Position auf dem Schaftrohr fixiert sind.

Fig. 7 zeigt die Ausführungsvariante in Fig. 6, bei einer darmwärts gerichteten, axialen Auslenkung des Schaftrohres innerhalb des dem Schaftrohr aufsitzenden, trans-anal platzierten Ballonkörpers um den Betrag C, wobei sich der Schaft innerhalb des Ballons in freier relativer Bewegung um einen Betrag C darmwärts verschieben lässt, ohne das es dabei zu einer, wie bei Fig. 4 beschriebenen, die Schaftbewegung begrenzenden elastischen Verformung der Hüllenanteile des intrarektalen Ballonsegmentes kommt.

Fig. 8 zeigt im schematischen Querschnitt eine Ausführung der Vorrichtung, bei der, das den Ballon tragende Schaftrohr durch eine schützend und/oder elastisch lumenaufrichtend wirkende Ummantelung ergänzt ist.

Fig. 9 zeigt im schematischen Querschnitt verschiedene Ausführungsvarianten einer Fig. 7 entsprechenden Ummantelung des Schaftrohres, mit vollständiger und teilweiser Segmentierung des Mantels zur Prädilektion des axialen Knick- bzw. Stauchungsverhaltens des Schaftes.

Fig. 10 zeigt weitere Ausführungsvarianten einer Ummantelung.

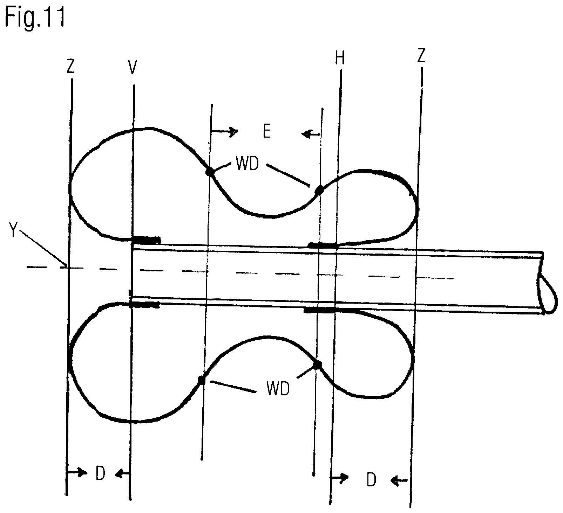

Fig. 11 zeigt eine besondere Ausführungsform der Vorrichtung zur sich selbsttätig anpassenden, axial gerichteten Dichtung des Ballons gegen die innere und äußere Öffnung des Analkanals, bei der die Ballonschaftenden beidseits um einen Betrag D auf dem Schaftrohr invertiert und auf dem Schaftrohr fixiert werden, welcher gewährleistet, dass das freie, distale Ende des Schaftrohres auch dann noch innerhalb des intra-rektalen Ballonsegmentes zu liegen kommt, wenn die sich die endständigen Ballonanteile des sanduhr- bzw. hantel-förmigen Ballonkörpers durch eine axiale Gegenrollbewegung maximal einander nähern.

Fig. 12 zeigt weitere Ausführungsformen des den Ballon tragenden Schaftkörpers.

Fig. 1 zeigt einen Längsschnitt durch ein erfindungsgemäßes Darmrohr 1, mit einem am distalen Ende eines Schaftrohres 6 aufgebrachten, sanduhr- oder hantelförmigen Ballonelement 2. Das Ballonelement weist an den Enden sphärische- oder diskoide Erweiterungen auf. Im mittigen Bereich zwischen den Erweiterungen befindet sich ein verjüngtes, beispielsweise zylindrisch oder annähernd zylindrisch ausgeformtes taillenartiges Segment 3. Das distale Ballonschaftende 9 ist bei dieser Ausführungsform derart auf einem Schaftrohr 6 aufgebracht, dass die Übergangspunkte 9e und 9f des unteren Ballonradius in den zylindrischen Teil des Ballonschaftes mit dem freien distalen Ende des Schaftrohres 6 bündig bzw. nahezu bündig abschließen. Die Verbindungslinie V der Punkte 9e und 9f wird im Folgenden als vordere Fixierungslinie bezeichnet und beschreibt die jeweils mechanisch wirksamen Fixierungspunkte des distalen Ballonendes auf dem den Ballon tragenden Schaftrohr. Das freie distale Ende des Schaftrohres liegt bei der beschriebenen Ausführung somit auf der vorderen Fixierungslinie V, welche nach distal wiederum von der Verbindungsline Z der Wendepunkte der Ballonschulterradien überragt wird, wodurch der nach distal überragende, frontale Anteil der intra-rektalen Ballonhülle einen unmittelbaren Kontakt von Darmanteilen mit dem freien Schaftende ausschließt und somit mechanisch puffernd wirkt.

Die Fixierung des proximalen Ballonschaftendes 10 auf dem Schaftrohr orientiert sich bei der beschriebenen Ausführungsform am Zustand der freien Entfaltung der Ballonhülle, bei zuvor bereits erfolgter Verbindung des distalen Ballonschaftes. Die proximale Verbindung erfolgt wahlweise auf der hinteren Fixierungslinie $H_1$ (bei nach distal invertiertem Ballonschaftende 10) oder auf der hinteren Fixierungslinie $H_2$ (bei nach proximal auslaufendem Ballonschaftende). Die hinteren Fixierungslinien konstruieren sich analog zur vorderen aus den Übergängen 10 und 10b der unteren Ballonschulterradien in den Ballonschaft und deren Verbindungslinie.

Fig. 2 zeigt in einem transversalen Schnitt die radiale Einstülpung 22 der Wandung des Schaftrohres 6, sowie die korrespondierende Faltung eines, in dieser Ausführungsform durchgängig fixiert auf dem Schaftrohr aufgebrachten Schaummantels 16. Potentiell schneidende Kantenformationen 23, wie sie sich im Scheitelbereich der Einstülpung 23 darstellen können, werden durch die aufliegende Schaumlage atraumatisch weich in ihrer Wirkung auf die anliegenden Gewebe gepolstert.

Für die Ausführung des Schaumkörpers werden bevorzugt weiche, sich elastisch aufrichtende Polyurethanschäume verwendet. Analog zur Unterstützung der axial wirkenden, elastischen Wiederaufrichtung der verformten Schaftrohrwandung, unterstützt eine elastisch wirkende Ummantelung die spontane radiale Aufrichtung einer radial eingefalteten Schaftrohrwandung.

Die Schaumelemente können bei der Herstellung der Vorrichtung ebenfalls direkt auf das tragende Rohr aufgeschäumt werden, wodurch sich eine flächige Verbindung mit der tragenden Unterlage durch Lösungsmittel oder Klebstoff erübrigt.

Vorteilhaft für den Materialverbund des Schaftkörpers haben sich, für sämtliche vorgestellten Ausführungsformen der Vorrichtung, beispielsweise Kombinationen folgender Materialien und Eigenschaften erwiesen. Das Schaftrohr 16 besteht aus einem Polyurethan der Shore Härte 70-90 A, weist einen Innendurchmesser von ca. 15 mm und eine Wandungsstärke von 0,2 bis 0,3 mm auf. Der Schaummantel 16 hat eine Wandstärke von ca. 1 bis 5 mm, vorzugsweise 1,5 bis 2,5 mm. Seine Konsistenz und Elastizität entspricht dem Material MS SuperSoft 70P, der Firma Filtrona Porus Technologies. Hierbei handelt es sich um einen hydrophilen PUR-Schaum auf MDI (MDI: Präpolymer-Typ) Basis.

Die beschriebene Kombination gestattet einen optimalen Aufrichtungseffekt des Rohrlumens sowie optimalen Schutz der anliegenden Körpergewebe.

Alternativ können auch weichere Schaumummantelungen verwendet werden, deren elastische Eigenschaften beispielsweise dem Material MS SuperSoft 60P entsprechen.

Härtere, weniger verformbare Schaumummantelungen lassen sich beispielsweise auf der Grundlage faserverstärkter Schäume, wie zum Beispiel der Qualität MS 70P-composite foam herstellen.

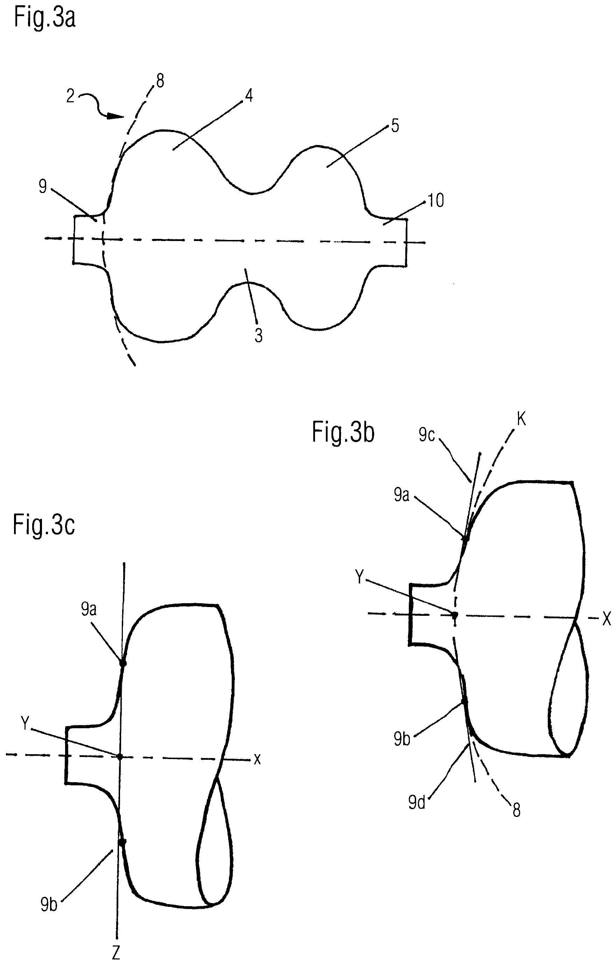

Die Figuren 3a bis 3c zeigen einen Längsschnitt durch einen frei entfalteten, bereits bei der Herstellung auf sein funktionelles Arbeitsmaß ausgeformten Ballonkörper 2, mit dessen freien Ballonschaftenden 9 und 10. Der vordere, frontal zum Darmlumen hin weisende Ballonradius 8 des intra-rektalen Ballonsegmentes ist als gestrichelte Linie angedeutet.

Der frontale Ballonradius 8 wird aus den beiden beiden Wendepunkten 9a und 9b sowie den beiden, diesen Punkten jeweils zugehörigen Tangenten 9c und 9d konstruiert. Hierzu wird ein Kreis konstruiert, welcher die beiden Wendepunkte 9a und 9b schneidet und dessen Tangenten (9c, 9d) in diesen Punkten denen der Wendepunkte entsprechen. Der Scheitelpunkt Y ergibt sich aus dem Schnittpunkt des Kreisumfangs mit der Symmetrieachse X des Ballons.

Für die im Folgenden verwendete Beschreibung des frontalen Scheitelpunktes der intra-rektalen Ballonerweiterung wird zur vereinfachten Darstellung des jeweils am weitesten nach distal reichenden Punktes der Ballonhülle der Schnittpunkt der Verbindungslinie Z der beiden Wendepunkte mit der Symmetrieachse X verwendet.

Der Ballonkörper verfügt über zwei, jeweils endständige sphärische oder annähernd sphärische Erweiterungen, wobei die distale, intra-rektale Ballonerweiterung 4 über ein mittiges, im Durchmesser verjüngtes bzw. tailliertes Segment 3 mit der proximalen, prä-analen Erweiterung 5 verbunden ist. Das mittige taillierte Segment 3 wird im Analkanal platziert bzw. nimmt die Strukturen des Anus zwischen den endständigen Ballonerweiterungen auf. Die mittige Verjüngung des Ballons gewährleistet zum einen eine, bei anwendungstypischen, axial auf die Vorrichtung wirksamen Zug- und Schubkräften weitgehend dislokationssichere Positionierung der Drainagevorrichtung in Analkanal und erlaubt zum anderen eine besonders großflächige und damit effiziente Dichtung der Ballonhülle gegen die Wandung bzw. Schleimhaut des Analkanals. Das mittige Segment erfüllt zudem eine schützende Funktion, indem der fortwährende direkte mechanische Kontakt der Strukturen des Rohrschaftes mit den empfindlichen Geweben des Analkanals, durch die protektiv wirkende Hülle des taillierten Zwischensegmentes verhindert wird. Die Wandungsstärke der Ballonhülle sollte im trans-analen Segment zur Erreichung einer guten Dichtungsleistung ca. 10 bis 50 $\mu$m, vorzugsweise jedoch nur 10 bis 20 $\mu$m betragen. Der Durchmesser im trans-analen Segment 3 sollte ca. 15 bis 45 mm, vorzugsweise 20 bis 30 mm aufweisen. Bedingt durch den relativ großen Durchmesser der trans-analen Ballonhülle, kommt es bei trans-analer Platzierung zu nach innen gerichteten radialen Einstülpungen der Ballonhülle. Durch die radiale Einstülpung der residualen Ballonhülle in diesem Abschnitt kann eine kraftintensive Aufdehnung der Hüllenwandung zur Dichtung des Analkanals zwischen Katheterschaft und Wandung des Analkanals vermieden werden.

Die vollständige Ausformung des taillierten Ballonkörpers bei der Ballonherstellung auf das funktionelle Arbeitsmaß vermeidet die bei Darmrohren mit konventionellen Ankerballonelementen erforderliche Aufdehnung einer in der Regel volumendehnbaren, nicht- oder nur teilweise ausgeformten Ballonhülle, unter hohem, unphysiologischen Druck, und ermöglicht im Gegensatz hierzu dauerhaft niedrig wirkende Ballonfülldrucke, die die Gewebedurchblutung im dem Ballon anliegenden Gewebe, nicht oder nur minimal beeinträchtigen.

Die Ballonkomponente wird vorzugsweise aus einem sehr dünnwandigen, nur wenig volumendehnbaren und insgesamt formstabilen Material mit geringer Herniationsneigung gefertigt, welches zwar im bevorzugten Wandstärkenbereich von wenigen Mikrometern die Eigenschaften einer körperverträglichen Weichfolie hat, sich bei steigendem Fülldruck jedoch nicht unkontrolliert verformt, und zudem bei von außen wirkendem Zug am Katheterschaft die Schlupfneigung des Ballons, wie sie bei hoch-volumendehnbaren Materialien wie z.B. Naturkautschuk oder Silikon zu erwarten wäre, begrenzt, und somit eine gute, volumendehnbaren Materialien deutlich überlegene trans-anale Ankerwirkung der Drainagevorrichtung ermöglicht. Mikro-dünnwandige Ballonfolien aus beispielsweise Polyurethanen der Härten Shore 70 A bis 95A, bevorzugt 85A bis 90A, gewährleisten eine erfindungsgemäß geforderte Hüllenstabilität. Die Wandungsstärke der Ballonhülle im Bereich ihrer endständigen Erweiterungen sollte ca. 7 bis 45 $\mu$m, vorzugsweise 10 bis 20 $\mu$m betragen.

Für die Formung entsprechend dünnwandiger Ballonfolien werden bevorzugt nicht oder nur gering volumendehnbare Materialien, wie z.B. Polyurethan (PUR), beispielsweise der Spezifikation Pellethane 2363 80A oder 90A, Dow Chemical Corp., oder auch Elastollan der Sorte 1180A bis 1195A, BASF AG, verwendet. Alternativ können auch Polyethylene, PVC, oder Mischungen aus den genannten Materialien, mit Polyurethan vergleichbaren oder auch geringeren elastischen Verformungseigenschaften verwendet werden.

Solche dünnwandigen, in komplexer Form ausgeformten Ballonfolien können bevorzugt durch Blasformung (hot-molding) aus zuvor extrudiertem Rohschlauchmaterial hergestellt werden, was durch die so erreichbare polymere Orientierung den geformten Ballonfolien eine außerordentliche Festigkeit verleiht. Erfindungsgemäße Ballonfolien können auch direkt aus der extrudierten, noch weichen, amorphen Schlauchmasse geformt werden (in-line molding).

Denkbar sind auch Tauchverfahren und eine entsprechende Fertigung aus flüssigen PVC oder PUR Materialien. Auch das Verschweißen von singulären Folienlagen zu Ballonkörpern ist vorstellbar.

Die Befüllung des Ballons erfolgt bevorzugt mit einem Füllvolumen, welches derart bemessen ist, dass es das Volumen des Ballons bei freier, nicht mit Druck beaufschlagter Entfaltung der dem Schaft aufsitzenden Ballonhülle unterschreitet. Eine derartige inkomplette Befüllung stellt sicher, dass die Ballonhülle bei der Befüllung durch den Anwender nicht bereits in den Zustand einer vollständigen, allseitigen Dehnung geht, und dabei Fülldruckwerte erreicht werden, die die intrarektal und trans-anal wirkenden physiologischen Kräfte überschreiten. Im Zustand der inkompletten Füllung der Ballonhülle wirkt lediglich die Summe der intra-rektal, der trans-anal und prä-anal auf den Ballon wirkenden Kräfte als allseitig homogene, trans-mural wirksame Kraft auf die dem Ballon exponierten Gewebe. Zur Sicherstellung einer adäquaten partiellen Füllung wird bevorzugt eine dem Produkt beigelegte Füllspritze mit entsprechender Volumenmarkierung verwendet.

Fig. 4 zeigt die in Fig. 1 beschriebene Vorrichtung in trans-analer Platzierung im Analkanal AK, im Zustand einer von proximal her, in das Darmlumen DL hinein gerichtete axialen Auslenkung des Katheterschaftes 6 innerhalb des dem Schaftrohr aufsitzenden Ballons um einen Betrag A, unter Wirkung der in Schaftlängsachse auf den Schaft anlastenden Kraft $F_1$.

Das Vordringen des distalen Schaftrohrendes 7 in das Darmlumen DL wird durch die, sich der freien Auslenkung des Schaftes entgegenwirkende, elastische Verformung der frontalen Ballonwandanteile des intra-rektalen Ballonsegmentes 4 begrenzt. Das distale Schaftrohrende bzw. die vordere Fixierungslinie V überschreitet bei der hier dargestellten Ausführung der Vorrichtung und wirkenden Kraft $F_1$, einen durch die elastischen Verformungseigenschaften der intrarektalen Ballonhülle vorgegebenen bzw. begrenzenden maximalen Auslenkungsweg A in das Darmlumen nicht.

Der proximale Bezugspunkt für die Bestimmung des Auslenkungsweges A ist die hintere Fixierungslinie H (H1oder auch H2) im unbelasteten, nicht mit einer Kraft $K_1$ anlastenden Zustand.

Bei erfindungsgemäßer Formgebung, Dimensionierung und Materialwahl der Ballonhülle soll der maximal mögliche Auslenkungsweg A des distalen Schaftrohrendes näherungsweise dem Scheitelpunkt S eines Radius entsprechen, welcher über dem größten Durchmesser GD der intra-rektalen Ballonerweiterung 4 errichtet wird. Der größte Durchmesser GD bezieht sich hierbei auf den jeweiligen Durchmesser im frei entfalteten, nicht mit Druck beaufschlagten Zustand des Ballons.

Der Ballonkörper 2 wird zur Gewährleistung einer entsprechenden Begrenzung der darmwärts relativ gerichteten Verformung der Ballonhülle bevorzugt aus einem Material mit geringer elastischer Verformbarkeit hergestellt, was insbesondere bei der bevorzugten dünnwandigen Ausführung der Ballonhülle im niedrigen Mikrometerbereich von besonderer Bedeutung ist, und typisch volumendehnbare Materialien wie z.B. Naturkautschuk oder Silikon für die Ballonherstellung weitgehend ausschließt.

Fig. 5 zeigt die in Fig. 1 und Fig. 4 beschriebene Vorrichtung im Zustand einer darmwärts gerichteten axialen Auslenkung des Katheterschaftes innerhalb eines trans-anal platzierten Ballons bei einer von proximal her, auf den Katheterschaft wirkenden Kraft $F_2$, wobei diese die in Fig. 4 beschriebene Kraft $F_1$ überschreitet.

Bei Überschreiten einer bestimmten kritischen Kraft $F_{knick}$ kommt es zu einer einfachen oder auch mehrfachen axialen Einknickung (KS) des Schaftrohres 6. Im Moment der Knickung bzw. Stauchung des Schaftrohres lässt der axial wirkende Widerstand des Schaftrohres gegen die von proximal auf den Schaft wirkende Kraft $K_2$ abrupt nach und die weitere Auslenkung des Schaftrohres bis auf den Gesamtweg B erfolgt bei nahezu konstanter Kraftwirkung des Schaftrohres auf die frontalen Anteile des intra-rektalen Ballonsegmentes.

Die maximale Auslenkung der vorderen Fixierungslinie V um den Betrag A wird im Verlauf der Auslenkung des Schaftes um den Betrag B jedoch nicht überschritten, was für die erfindungsgemäße sichere Fixierung des freien, potentiell perforationsgefährdenden Schaftendes 7 entscheidend ist.

Der proximale Bezugspunkt für die Bestimmung des Auslenkungsweges B ist wiederum die hintere Fixierungslinie H (H1 oder auch H2) im unbelasteten, nicht mit einer Kraft K2 anlastenden Zustand.

In entsprechender Weise kommt es umgekehrt, bei einer von distal nach proximal gerichteten Krafteinwirkung auf das Schaftrohr initial zu einer nach proximal gerichteten Auslenkung des Schaftkörpers innerhalb des trans-anal platzierten Ballons, und zu einer entsprechenden Verformung der proximalen Hüllenanteile des prä-analen Ballonsegmentes 5. Mit zunehmender Kraftwirkung und Überschreitung einer kritischen Kraft geht das Schaftrohr 6 auch in diesem Auslenkungsfall in den zuvor beschriebenen eingestauchten Zustand KS über und lässt eine weitere Auslenkung des Schaftes nach proximal bei dann konstanter auf den Schaft wirkender Kraft zu.

Ein derartiges Knickungs- bzw. Stauchungsverhalten der Vorrichtung ist vor allem dann relevant, wenn sich Darmanteile von distal her auf die frontalen Ballonanteile auflegen oder es im Rahmen einer forcierten Auslenkung des Schaftrohres in den Darm hinein und zu einer völligen Dislokation der Vorrichtung aus seiner transanalen Fixierung kommt, und die intra-rektalen Anteile der Vorrichtung auf die Darmwandung aufschlagen.

Die kritische Kraft $K_{knick}$ sollte bei den jeweiligen Ausführungsformen der vorliegenden Erfindung vorzugsweise bei einer von proximal her, axial auf den Schaft lastenden Gewichtskraft von ca. 150 bis 1000 Gramm erreicht sein, besonders bevorzugt jedoch bei Kräften im Bereich von 200 bis 500 Gramm.

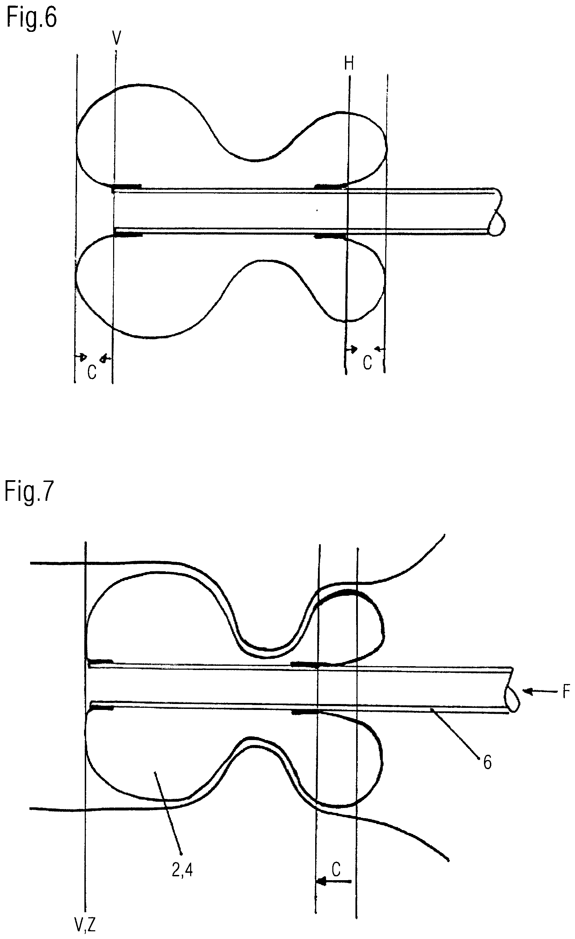

Wie Fig. 6 zeigt, schlägt die Erfindung neben der besonderen Berücksichtigung länger-streckiger Auslenkungen des Schaftrohres innerhalb des trans-anal platzierten Ballons ebenfalls Ausführungsformen vor, bei denen die vordere und hintere Fixierungslinie V und H der Ballonschaftenden, auf dem Schaftrohr jeweils um einen bestimmten Betrag C zueinander hin verschoben (invertiert) wird, wodurch kleinere axiale Auslenkungen des Katheterschaftes über den Weg C durch eine axiale Relativbewegung zwischen Ballon und Schaft gepuffert werden können, ohne das es zu einer Verformung der Hülle des intra-rektalen Ballonsegmentes kommt bzw. sich eine distale Verschiebung der vorderen Fixierungslinie V über den Ballonradius 8 hinaus einstellt. Weiter reichende Auslenkungen des Schaftes, welche den durch die Inversion der Fixierungsenden ermöglichten puffernden Weg C überschreiten, werden in analoger Weise zur Ausführung in Abb.3 durch eine elastische Hüllendehnung dämpfend abgefangen.

Bei einem äußeren Schaftdurchmesser von ca. 12 mm, sollte der nach innen gerichtete Versatz VF der Fixierungslinien V und H bevorzugt ca. 10 bis 12 mm betragen. Bei einem 15 mm messenden Schaftaußendurchmesser sollte der Versatz VF entsprechend 12 bis 16 mm betragen. Der Bezugspunkt für den Versatz sind die jeweiligen Verbindungslinien Z zwischen den Wendepunkten der Radien in den Ballonschultern des frei entfalteten, nicht mit Druck beaufschlagten Ballons.

Fig. 7 zeigt die in Fig. 6 beschriebene Vorrichtung im trans-anal platzierten Zustand, wobei sich das den Ballon tragende Schaftrohr innerhalb des transanal platzierten Ballons relativ frei um einen Betrag C darmwärts verschieben lässt, ohne das es dabei zu einer, die Schaftbewegung begrenzenden, elastischen Verformung der Hüllenanteile des intra-rektalen Ballonsegmentes kommt.

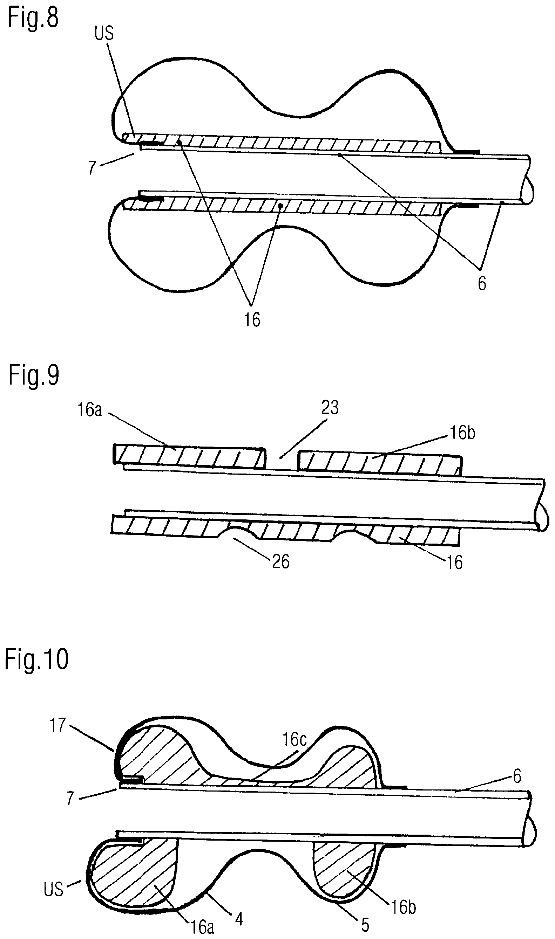

Fig. 8 stellt besondere Ausführungsformen der Vorrichtung vor, bei denen das den Ballon 2 tragende Schaftrohr 6 durch eine schützende und/oder elastisch lumenaufrichtend wirkende Ummantelung 16 ergänzt ist. Die Abbildung zeigt einen Längsschnitt durch ein Darmrohr 1 mit einer, im Balloninneren auf dem Schaftelement 6 aufgebrachten, durchgängigen Ummantelung aus vorzugsweise schaumartigen, elastisch verformbaren und sich spontan wiederaufrichtenden Materialien.

Die Ummantelung 16 reicht bevorzugt über das distale Ende des Schaftrohres 6 hinaus (Überstand US) und ermöglicht so eine weiteren puffernden Schutz der Darmwand gegenüber dem freien Ende 7 des Schaftrohres. Die Schaumkörper überragen dabei das distale Ende des Schaftrohres um vorzugsweise 3 bis 12 mm, und besonders vorteilhaft um 4 bis 8 mm.

Optional kann die Ummantelung 16 auch auf den, sich nach proximal an das präanale Schaftsegment anschließendem Schaftrohrabschnitt ausdehnen.

Fig. 9 In dieser Ausführungsform der Vorrichtung wird eine Segmentierung des ummantelnden Körpers 16 vorgeschlagen. Diese erfolgt vorzugsweise innerhalb des intra-rektalen Ballonsegmentes oder alternativ im Übergangsbereich zwischen diesem Segment und dem trans-analen Segment. Der ummantelnde Körper kann auf dem Drainagerohr als vollständig getrennte und voneinander beabstandete Einheiten, z.B. 16a und 16b aufgebracht sein. Alternativ kann eine Segmentierung durch eine ausgeprägte Taillierung 26 einer durchgehenden Mantelstruktur 16 erfolgen. Die Segmentierung bzw. Taillierung reduziert die Knickstabilität des Schaftkörpers im Zwischensegmentbereich 23 bzw. Bereich der Mantelverjüngung 26, und präformiert daher dessen axiales Einknicken in einem gewünschten Schaftabschnitt (Sollknickstelle). Es können mehrere derartige Knickstellen im Verlauf der Ummantelung des Drainagerohres 6 angeordnet sein.

Bei einem Innendurchmesser des Schaftrohres von ca. 15 mm sollte das schaumfreie Stück 23 zwischen den Schaumelementen 16a und 16b vorzugsweise eine Länge von 5 bis 15 mm, besonders bevorzugt von 5 bis 10 mm aufweisen.

Fig. 10 zeigt das distale Schaumsegment 16a innerhalb des Ballonsegmentes 4, welches beispielsweise in Kugel- oder Diskusform, mit der Funktion einer Widerlagerkomponente ausgebildet sein kann. Die Komponente sorgt für einen zusätzlichen Ankereffekt beim Einführen des Rohres in das Rektum. Die Widerlagerkomponente verhindert dabei das ungewollte Herausgleiten des Rohres vor der Befüllung des Ballons. Entsprechend ausgeformt, kann ein Schaumsegment 16b, welches dem Schaftrohr 6 innerhalb des prä-analen Segmentes 5 aufsitzt, als orientierende Marke für die Feststellung der korrekten Einführtiefe der Vorrichtung in den Anus dienen.

Ein entsprechend geformtes distales Segment 16a kann zudem eine stützende Wirkung auf das Schaftrohr haben und das seitliche Abkippen des Schaftes zur Darmwandung hin einschränken. Diese Wirkung ist dann umso zuverlässiger, je weiter distal, zur Schaftöffnung 7 hin der Widerlagerkörper auf dem Schaft aufgebracht ist. Der Schaumkörper besteht bevorzugt aus einem Polyurethanschaum, der wiederum bevorzugt, viskoelastische Verformungs- und Aufrichtungseigenschaften hat.

Das distale und proximale Schaumsegment kann zudem durch ein verjüngtes Zwischensegment durchgängig verbunden werden, wodurch sich der Schaumkörper 16 als Einheit in der Form einer Sanduhr oder Hantel darstellt.

Zur verbesserten Schutz vor axialen Auslenkungen des distalen Rohrendes in den Darm hinein kann eine strukturelle Verbindung 17 der distalen Ballonhülle mit der nach distal gerichteten Fläche des innenliegenden Schaumkörpers 16a bzw. der entsprechenden Fläche des oben beschriebenen hantel-förmigen Schaumkörpers erfolgen. Die Verbindung 17 kann beispielsweise durch flächige Verklebung hergestellt werden.

Analog zur Ausführung in Fig. 8 kann auch hier das distale Segment 16a bzw. das distale Ende der Ummantelung 16 um einen bestimmten überstehenden Versatz US über das distale freie Ende 7 des Schaftrohres hinausreichen.

Fig. 11 beschreibt eine weitere Möglichkeit der atraumatischen Sicherung des distalen Katheterschaftendes bei Drainagen mit erfindungsgemäß taillierten Ballonkomponenten, wobei die Fixierungslinien V und H derart zueinander hin versetzt werden, dass sich eine ausgeprägte Gegenrollbewegung der endständigen Ballonsegmente einstellt. Der Betrag des beidseitigen Versatzes D der Fixierungslinien V und H soll dabei in Summe kleiner/gleich der Distanz E zwischen den Wendepunkten WD der Schulterradien der distalen Ballonerweiterung und WP der proximalen Erweiterung sein. Der Versatz D bezieht sich, analog zu dessen Bestimmung in den vorausgehenden Abbildungen, auf die Scheitelpunkte Z der vorderen und hinteren Ballonradien.

Die so ermöglichte axiale Gegenrollung der Ballonerweiterungen 4 und 5 auf den äußeren und inneren Anus hin, soll es ermöglichen, auch bei einem anatomisch von der Norm abweichenden, verkürzten Analkanal eine dislokationsstabile und gut dichtende Platzierung der Drainage im Anus zu ermöglichen. Das freie distale Ende 7 des Schaftrohres wird hierbei wiederum bevorzugt in Deckung mit der vorderen Fixierungslinie V des distalen Ballonschaftendes 9 verbunden.

Bei einer von proximal in axialer Richtung auf den Schaft wirkenden Kraft, bleibt das Schaftrohrende 7, analog zur Ausführungsform in Fig. 4, bei erfindungsgemäßer Ausführung der Ballonhülle und des Schaftrohres ebenfalls in seiner maximalen Auslenkung innerhalb des trans-anal platzierten Balloons derart begrenzt, dass ein definierter Scheitelpunkt S vom freien Schaftrohrende nicht nach überschritten wird.

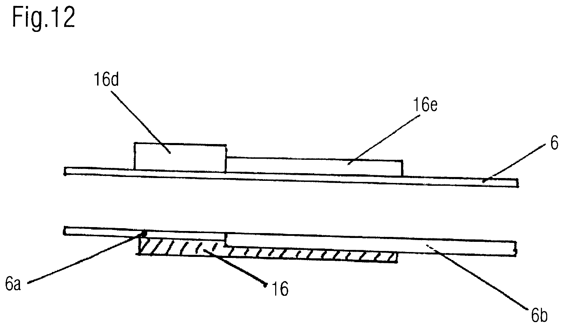

Fig. 12 beschreibt eine alternative Ausführungsform der Komponenten des erfindungsgemäßen Schaftkörpers (Schaftrohr und Ummantelung), wobei das Gestaltungsprinzips der axialen Stauchung und radialen Faltung des Schaftrohres 6 durch Segmentierung des Schaftes in Abschnitte von Materialien verschiedener Härte, Wandungsstärke und Verformungseigenschaften aufgebaut sein kann.

Beispielsweise kann das trans-anale oder auch das prä-anale bzw. sich daran nach proximal anschließende Segment des Schaftes aus relativ hartem, geringer verformbaren Material gefertigt sein, wie das intra-rektale Segment des Schaftes. Der beschriebene Stauchungs- und Faltungseffekt lässt sich so auf einzelne Segmente beschränken, während andere weitgehend lumenstabil sind und der reversiblen elastischen Verformung des Schaftes entgegenwirken.

Neben der Verwendung elastisch verformbarer Materialien, sind auch nicht elastisch, plastisch wirkende Schaftsegmente, aus beispielsweise PVC oder PUR/PVC-Mischungen denkbar.

Die Befüllung des der vorgestellten Ballonelemente erfolgt bevorzugt durch eine in die Wandung des Schaftrohres integrierte Füll-Leitung. Es wird bevorzugt ein kompressibles gasförmiges Medium, wie Luft, verwendet.

Zur volumenkontrollierten Befüllung wird bevorzugt ein beigefügtes Spritzenelement verwendet, welches das jeweilige Füllvolumen durch eine entsprechende Markierung auf dem Spritzenkörper vorgibt.

Die Befüllung des Ballons erfolgt bevorzugt inkomplett, mit einem Füllvolumen, welches dem jeweiligen Volumen entspricht, welches der dem Schaft aufsitzende, frei entfaltete, nicht mit Druck beaufschlagte Ballon aufnimmt, abzüglich des entsprechenden, vom trans-analen Ballonsegment aufgenommenen Volumens. Das trans-anale Ballonsegment definiert sich hierbei als der Ballonteil zwischen den Wendepunkten WD und WP (siehe Fig. 11).

Besonders vorteilhaft zur Erreichung eines optimal niedrigen Fülldruckes bei gleichzeitiger ausreichender Anker- und Dichtungsfunktion des Ballons ist eine Befüllung mit einem Füllvolumen, welches dem zuvor ermittelten Füllvolumen entspricht, aber um ca. 10 bis 30 Prozent des vom trans-analen Ballonsegment aufgenommenen Volumens vergrößert ist.

An einem konkreten Ausführungsbeispiel wird ferner das elastische Faltungs- und Aufrichtungsverhalten eines schaumummantelten Schaftrohres bei axial und radial anlastender Kraft beschrieben.

Das Schaftrohr 6 wird wie im Folgenden ausgeführt:

- Elastollan 1180A (der Firma BASF)
- Innendurchmesser 15 mm
- Wandungsstärke 0,3 mm

Das Schaftrohr wird mit einem hantel-förmigen Ballon 2 verbunden, wobei dieser durch eine Füllleitung mit wechselnden Fülldrucken beaufschlagt wird. Die Schaumummantelung 16 ist innerhalb des Ballons durchgängig auf der Außenfläche des Schaftrohres aufgebracht und weist eine Wandstärke von 2 mm auf. Das Schaummaterial entspricht der Spezifikation des Typs MS SuperSoft 70P, der Firma Filtrona Porus Technologies.

In der folgenden Tabelle wird die sich jeweils einstellende maximale Einengung des Schaftrohrlumens (Drainagelumen) bei einem bestimmten, dem Schaftkörper innerhalb des Ballons allseitig anlastenden Fülldruck dargestellt. Es wird der jeweils kleinste transversale Abstand zwischen den sich nähernden Rohrinnenwandungen als Drainagelumen-Maß DM, im den Ballon tragenden Abschnitt des Schaftes, festgestellt.

Tabelle: Beziehung Fülldruck/Drainagelumen bei konkreter, beispielhafter Ausführung eines schaumummantelten Schaftrohres

| Ballonfülldruck (mbar) | Drainagelumen-Maß DM (mm) |
|---|---|
| | |
| 0 | 14 |
| 5 | 12-14 |
| 10 | 9-11 |
| 15 | 6-9 |
| 20 | 5-8 |
| 25 | 4-6 |
| 30 | 3-5 |
| 35 | 1-3 |
| 40 | 0-2 |
| 45 | 0 |

Die axiale Knickung des Schaftrohres bei einer von über die Schaftlängsachse auf den Schaft einwirkenden Kraft $F_{knick}$, erfolgt bei dieser konkreten Ausführung der Vorrichtung bei einer im Bereich der hintere Fixierungslinie H anlastenden Gewichtskraft von ca. 300 bis 350 Gramm.

Die Erfindung schlägt auch einfache Ausführungsformen der Drainagevorrichtung vor, die über keine Ummantelung verfügen und lediglich ein Schaftrohr als den Ballon tragender Körper aufweisen. Die folgende Tabelle gibt zum Vergleich das Verformungsverhalten eines bau-gleichen, Schaftrohres ohne Ummantelung wieder.

Tabelle: Beziehung Fülldruck/Drainagelumen bei konkreter, beispielhafter Ausführung eines nichtummantelten Schaftrohres

| Ballonfülldruck (mbar) | Drainagelumen DM (mm) |
|---|---|
|  |  |
| 0 | 14 |
| 5 | 8-12 |
| 10 | 5-8 |
| 15 | 2-4 |
| 20 | 1-2 |
| 25 | 0 |

Die axiale Knickung des Schaftrohres bei einer von über die Schaftlängsachse auf den Schaft einwirkenden Kraft $F_{knick}$, erfolgt bei dieser konkreten Ausführung der Vorrichtung bei einer im Bereich der hintere Fixierungslinie H anlastenden Gewichtskraft von ca. 200 bis 250 Gramm.

Neben der trans-analen Anwendung der Vorrichtung, können erfindungsgemäß ausgeführte Darmrohre auch für die perforationssichere Platzierung in transabdominellen, chirurgisch angelegten Stomata/Öffnungen zum Darm oder weiteren natürlichen Körperöffnungen eingeführt und platziert werden.

Die beschriebene Vorrichtung kann in den hier vorgeschlagenen Ausführungen ebenfalls als trans-analer Einlaufkatheter oder im Rahmen einer postoperativen trans-analen Tamponade nach Anal- oder Rektumchirurgie zur Anwendung kommen. Besonders vorteilhaft erweisen sich hier die trans-anale Dichtungsfunktion durch das mittige, taillierte Ballonsegment sowie die atraumatische Positionierung der Schaftspitze auch bei akzidenteller, forcierter axialer des Schaftes in das Darmlumen, sowie die Verwendung zusätzlicher Widerlager- bzw. Schaumkörper in der intra-rektalen und/oder prä-analen Erweiterung der Ballonkomponente. Die Erfindung ist durch die folgenden Ansprüche definiert.

**Patentansprüche**

1. Vorrichtung zur trans-analen Drainage von Stuhl aus dem Rektum eines Patienten durch ein katheterartiges Element mit einem rohrförmigen Schaftkörper (6) und einem aufblasbaren Ballonelement (2), und/oder zur trans-analen Applikation von Einlaufflüssigkeit durch ein katheterartiges Element mit einem rohrförmigen Schaftkörper (6) und einem aufblasbaren Ballonelement (2), der aus einem dünnwandigen Weichfolienmaterial von begrenzter elastischer Verformbarkeit unter Präformierung mit einer taillierten oder hantelförmigen oder sanduhrförmigen Gestalt hergestellt ist, mit zwei endständigen Ballonabschnitten von größerem Schlauchradius ($D_1/2$, $D_2/2$) sowie einem dazwischen angeordneten, mittigen, demgegenüber verjüngten Ballonabschnitt von verringertem Schlauchradius, welcher trans-anal platziert wird, derart, dass der distal anschließende, radial erweiterte Ballonabschnitt intra-rektal platziert ist und der proximal anschließende, radial erweiterte Ballonabschnitt extra-korporal, **dadurch gekennzeichnet, dass** die beiden Ballonenden sich verjüngen und in einem axialen Abstand voneinander auf der Mantelfläche des Schaftkörpers (6) fixiert sind, wobei der intra-rektale Ballonabschnitt in kräftefreiem Zustand den distalen Bereich des Schaftkörpers (6) vollständig umgibt und derart bemessen ist, dass bei einer darmwärts gerichteten axialen Auslenkung des Schaftkörpers (6) infolge der damit verbundenen Verformung der das Schaftende umgebenden Ballonhülle eine entgegengesetzt wirkende Kraft erzeugt wird, welche das distale Ende des Schaftkörpers (6) in seiner Bewegung nach distal dämpft und/oder begrenzt, und wobei der Schaftkörper (6) derartige elastische Verformbarkeits- und Wiederaufrichtungseigennschaften aufweist, dass er bei normalem anlastenden analen Schließmuskeltonus in einen radial eingefalteten Zustand übergeht oder auf ein im Durchmesser kleineres Maß kollabiert, bei Nachlassen des Schließmuskeltonus sich jedoch spontan wieder auf sein ursprüngliches Durchmessermaß aufrichtet.

2. Vorrichtung nach Anspruch 1, **dadurch gekennzeichnet, dass** der Katheterschaft derart ausgebildet ist, dass er beim Kollabieren radiale Einstülpungen (22) ausbildet.

3. Vorrichtung nach einem der vorhergehenden Ansprüche, **dadurch gekennzeichnet, dass** die elastischen Verformungs- und/oder Wiederaufrichtungseigenschaften des Schaftkörpers und/oder einer darauf applizierten Umhüllung derart eingestellt sind, dass bei Fülldrucken bis 25 mbar das drainierende Lumen des Schaftrohres bei freier Entfaltung des Ballons außerhalb des Körpers im Bereich seiner größten Verengung auf nicht mehr als 50 % seines frei entfalteten Ausgangsmaßes reduziert ist, und/oder bei Fülldrucken bis 45 mbar auf nicht mehr als 20% reduziert ist.

4.  Vorrichtung nach nach einem der vorhergehenden Ansprüche, **dadurch gekennzeichnet, dass** der intra-rektale Ballonabschnitt und der extra-korporale Ballonabschnitt etwa gleiche Längs-Querschnitte aufweisen.

5.  Vorrichtung nach einem der vorhergehenden Ansprüche, **dadurch gekennzeichnet, dass** der Schaftkörper mit einer Ummantelung versehen ist.

6.  Vorrichtung nach Anspruch 5, **dadurch gekennzeichnet, dass** die Ummantelung die Form von Schaumelementen aufweist.

7.  Vorrichtung nach Anspruch 6, **dadurch gekennzeichnet, dass** die Ummantelung, insbesondere ein Schaumeiement, über das distale Katheterende hinausreicht, vorzugsweise um wenigstens 5 mm, insbesondere um 10 mm oder weniger.

8.  Vorrichtung nach Anspruch 5 bis 7, **dadurch gekennzeichnet, dass** die Ummantelung segmentiert ist und/oder Querschnittsveränderungen und/oder Ausnehmungen aufweist.

9.  Vorrichtung nach Anspruch 5 bis 8, **dadurch gekennzeichnet, dass** die Ummantelung außen von dem Ballon ganz oder teilweise umgeben wird.

10. Vorrichtung nach Anspruch 9, **dadurch gekennzeichnet, dass** die Ummantelung an der Innenseite des Ballons fixiert ist, insbesondere an der Innenseite des intra-rektalen Ballonabschnitts.

11. Vorrichtung nach einem der vorhergehenden Ansprüche, dass das distale Ende des Katheterschaftes nicht aus der das Schaftende umgebenden, distalen bzw. intra-rektalen Ballonerweiterung in einer den Darm potentiell traumatisierenden Weise hervortreten kann, wobei die distale Spitze des Kathetersschaftes in dessen nicht ausgelenkten Ruhestand vollständig proximal einer von der Symmetrieachse (X) lotrecht durchsetzten Ebene (Z) zu liegen kommt, welche den intra-rektalen Ballonabschnitt vollständig von distal tangiert.

12. Vorrichtung nach Anspruch 1 oder 2, **dadurch gekennzeichnet, dass** bei weitergehender, darmwärts gerichteter, axialer Auslenkung des proximalen Schaftendes der den Ballon tragende Schaft in den Zustand einer einfachen oder mehrfachen axialen Stauchung übergeht, insbesondere bei längerstreckigen darmwärts gerichteten Bewegungen des Schaftes, oder bei einer vollständigen Dislokation des gesamten ballontragenden Anteils der Drainage aus der trans-analen Platzierung heraus in den Darm hinein, oder bei einer forcierten Kollision der vorderen Drainageanteile mit Anteilen der Darmwandung.

13. Vorrichtung nach einem der vorhergehenden Ansprüche, **gekennzeichnet durch** einen Toleranzbereich mit einfachem, relativen Spiel zwischen Katheterschaft und Ballon, d.h., mit einer minimalen, rückstellenden Kraft $F_r \approx 0$, bei einer Auslenkung x gegenüber dem neutralen, kräftefreien Zustand um einen Wert bis zu einem ersten Grenzwert $G_1$:

$$x \leq G_1 \Rightarrow F_r = 0 \pm \varepsilon,$$

wobei $|\varepsilon| \leq 10$ g, vorzugsweise $|\varepsilon| \leq 5$ g, insbesondere $|\varepsilon| \leq 2$ g.

14. Vorrichtung nach einem der vorhergehenden Ansprüche, **gekennzeichnet durch** eine Rückstellkraft durch Verformung des intrarektalen Ballonabschnitts, d.h., mit einer rückstellenden Kraft $F_r$ der Ballonhülle, bei einer Auslenkung x gegenüber dem neutralen, kräftefreien Zustand um einen Wert zwischen einem ersten Grenzwert $G_1$ und einem zweiten Grenzwert $G_2$:

$$G_1 \leq x \leq G_2 => F_r \approx c_H * (x - G_1),$$

wobei $c_H$ der Federkonstante der drucklos aufgeblähten Ballonhülle entspricht.

15. Vorrichtung nach einem der vorhergehenden Ansprüche, **gekennzeichnet durch** eine Rückstellkraft durch Verformung (z.B. axiale Knickung oder Stauchung) des Katheterschaftes, d.h., mit einer rückstellenden Kraft $F_r$ des Katheterschaftes, bei einer Auslenkung x gegenüber dem neutralen, kräftefreien Zustand um einen Wert oberhalb des zweiten Grenzwerts $G_2$.

$$G_2 \leq x => F_r = c_K * (x - G_2),$$

wobei $c_K$ der (kleinsten) Federkonstante des Katheterschafts entspricht.

## Claims

1. Device for transanal drainage of stool from the rectum of a patient through a catheter-like element comprising a tubular shaft body (6) and an inflatable balloon element (2), and/or for the transanal administration of an enema fluid through a catheter-like element comprising a tubular shaft body (6) and an inflatable balloon element (2) produced from a thin-walled soft film material of limited elastic deformability under preformation with a waisted shape, having two terminal balloon sections with a larger tubular radius ($D_1/2$, $D_2/2$) as well as, disposed therebetween, and tapered relative thereto, a middle balloon section provided with a reduced tubular radius, which is placed transanally, such that the distally adjoining radially enlarged balloon section is placed intrarectally and the proximally adjoining radially enlarged balloon section extracorporeally, **characterized in that** the two balloon ends taper, and are fixed at an axial distance from each other, on a lateral surface of the shaft body (6), wherein, in the force-free state, the intrarectal balloon section completely surrounds the distal region of the shaft body (6) and is dimensioned such that in the presence of an axial displacement of the shaft body (6) towards the bowel, all attendant deformation of the balloon envelope surrounding the shaft end operates to generate an oppositely acting force that damps and/or limits the movement of the distal end of the shaft body (6) in a distal direction, an wherein the elastic deforming and/or restraightening properties of the shaft body (6) are such that in the presence of a normal sphincter muscle tone bearing against it from the outside, the shaft body (6) transitions into a radially invaginate state or collapses to a size smaller in diameter, but, upon relaxation of the tone of the sphincter muscle, spontaneously erects to its original diameter.

2. Device according to one of the preceding claims, **characterized in that** the catheter shaft is constructed in such way that it forms radial invaginations (22) while collapsing.

3. Device according to one of the preceding claims, **characterized in that** the elastic deforming and/or restraightening properties of the shaft body, and/or of an envelope applied thereto, are adjusted in such way that under filling pressures of up to 25 mbar, the draining lumen of the shaft tube, with the balloon freely unfolded outside the body, is reduced in a region of its greatest narrowing to no more than 50% of its freely unfolded initial dimension, and/or under filling pressures of up to 45 mbar is reduced to no more than 20%.

4. Device according to one of the preceding claims, **characterized in that** the intrarectal balloon section and the extracorporeal balloon section exhibit substantially identical longitudinal cross sections.

5. Device according to one of the preceding claims, **characterized in that** the shaft body is provided with a sheath.

6. Device according to claim 5, **characterized in that** the sheath has the form of foam elements.

7. Device according to claim 6, **characterized in that** the sheath, especially a foam element, extends beyond the distal end of the catheter, preferably by at least 5 mm, especially by 10 mm or less.

8. Device according to one of claims 5 to 7, **characterized in that** the sheath is segmented, and/or is provided with changes in cross section and/or with recesses.

9.  Device according to one of claims 5 to 8, **characterized in that** the sheath is entirely or partially surrounded by the balloon at its outside.

10. Device according to claim 9, **characterized in that** the sheath is fixed to the inner face of the balloon, especially to the inner face of the intrarectal balloon section.

11. Device according to one of the preceding claims, **characterized in that** the distal end of the catheter shaft cannot protrude from the distal or, respectively, intrarectal balloon extension surrounding the end of the shaft in a manner potentially traumatizing the bowel, wherein, in its non-displaced state of rest, the distal tip of the catheter shaft is situated entirely proximally to a plane (Z) penetrated perpendicularly by the axis of symmetry (X) and being tangent to the intrarectal balloon section vompletely from the distal side.

12. Device according to one of the preceding claims, **characterized in that** in the presence of a greater axial displacement of the proximal shaft end towards the bowel, the shaft supporting the balloon transitions into a state of a single or multiple axial buckling, especially at at longer distance movements of the shaft towards the bowel, or at a complete dislocation of the entire portion of the drainage, which is supporting the balloon, from its transanal placement into the bowel, or at a force dollision of the front portions of the drainage with portions of the intestinal wall.

13. Device according to one of the preceding claims, **characterized in that** there is provided a tolerance range with relative play between the catheter shaft and the balloon, with a minimal restoring force $F_r \approx 0$, in the presence of a displacement x from the neutral, force-free state by a value up to a first limit value $G_1$:

$$ x \leq G_1 => F_r = 0 \pm \varepsilon, $$

where $|\varepsilon| \leq 10$ g, preferably $|\varepsilon| \leq 5$ g, particularly $|\varepsilon| \leq 2$g.

14. Device according to one of the preceding claims, **characterized in that** the restoring force due to deformation of the intrarectal balloon section, i.e., with the restoring force $F_r$ of the balloon envelope, in the presence of a displacement x from the neutral, force-free state by a value between the first limit value $G_1$ and a second limit value $G_2$:

$$ G_1 \leq x \leq G_2 => F_r \approx c_H * (x - G_1), $$

where $c_H$ represents the spring constant of the balloon envelope inflated without pressure.

15. Device according to one of the preceding claims, **characterized in that** the restoring force due to deformation (e.g. axial kinking or buckling) of the catheter shaft, i.e., with the restoring force $F_r$ of the catheter shaft, in the presence of a displacement x from the neutral, force-free state by a value between above the second limit value $G_2$:

$$ G_2 \leq x => F_r = c_K * (x - G_2), $$

wherein represents the smallest spring constant of the catheter shaft.

**Revendications**

1.  Dispositif de drainage transanal des selles hors du rectum d'un patient au moyen d'un élément de type cathéter avec un corps de tige tubulaire (6) et un élément de ballonnet (2) gonflable, et/ou d'application transanale de liquide de lavement au moyen d'un élément de type cathéter comportant un corps de tige tubulaire (6) et un élément de ballonnet (2) gonflable qui est réalisé en un matériau en feuille souple à paroi mince de déformabilité élastique limitée, auquel a été conférée une forme cintrée ou en forme d'haltère ou de sablier, par préfaçonnage lors de la fabrication, ledit ballonnet présentant deux parties terminales de plus grand rayon de tuyau flexible ($D_1/2$ ; $D_2/2$) ainsi qu'une partie de ballonnet centrale, interposée, amincie par rapport à celui-ci de plus petit rayon de tuyau de flexible, laquelle est placée de manière transanale de sorte que la partie de ballonnet distalement adjacente et radialement étendue soit placée à l'intérieur du rectum et la partie de ballonnet proximalement adjacente et radialement étendue soit placée à l'extérieur du corps, **caractérisé en ce que** les deux extrémités de ballonnet s'amincissent et sont fixées à une distance axiale l'une de l'autre sur la surface périphérique du corps de tige (6), **en ce que** la partie de ballonnet à l'intérieur du rectum à l'état sans exercice de force entoure complètement la zone distale du corps de tige (6) et est dimensionnée de sorte que, lors d'une déviation axiale dirigée vers l'intestin du corps de tige (6) suite à la déformation qui y est liée de l'enveloppe du ballonnet entourant l'extrémité de tige, une force agissant en sens opposé soit générée, laquelle atténue distalement et/ou limite l'extrémité distale du corps de tige (6) dans son déplacement, et **en ce que** le corps de tige (6) présente de telles propriétés de déformabilité élastiques et de redressement qu'il passe à un état radialement replié avec un tonus du sphincter anal sous l'effet d'une pression normale ou s'affaisse à une dimension de plus petit diamètre, lors de la diminution du tonus du sphincter se redresse toutefois spontanément à sa dimension diamétrale initiale.

2. Dispositif selon l'une des revendications précédentes, **caractérisé en ce que** la tige de cathéter est telle qu'elle forme des retournements radiaux (22) lors de l'affaissement.

3. Dispositif selon l'une des revendications précédentes, **caractérisé en ce que** les propriétés de déformation élastiques et/ou de redressement du corps de tige et/ou d'une enveloppe qui y est appliquée sont prédéfinies de façon à ce que jusqu'à une pression de remplissage de 25 mbar la lumière drainante du tube de tige au déploiement libre du ballonnet hors du corps dans la zone de son plus grand rétrécissement ne soit pas réduite à plus de 50% de sa dimension de sortie librement déployée, et/ou jusqu'à une pression de 45 mbar à plus de 20%.

4. Dispositif selon l'une des revendications précédentes, **caractérisé en ce que** la partie de ballonnet intrarectale et la partie de ballonnet extracorporelle ont approximativement des sections transversales longitudinales égales.

5. Dispositif selon l'une des revendications précédentes, **caractérisé en ce que** le corps de tige est pourvu d'une gaine.

6. Dispositif selon la revendication 5, **caractérisé en ce que** la gaine a la forme d'éléments en mousse.

7. Dispositif selon la revendication 6, **caractérisé en ce que** la gaine, en particulier un élément en mousse, dépasse l'extrémité de cathéter distale, de préférence d'au moins 5 mm, en particulier de 10 mm ou moins.

8. Dispositif selon la revendication 5 à 7, **caractérisé en ce que** la gaine est segmentée et/ou présente des modifications de section transversale et/ou des évidements.

9. Dispositif selon la revendication 5 à 8, **caractérisé en ce que** la gaine de l'extérieur est entourée entièrement ou partiellement par le ballonnet.

10. Dispositif selon la revendication 9, **caractérisé en ce que** la gaine est fixée sur la face intérieure du ballonnet, en particulier sur la face intérieure de la partie de ballonnet intrarectale.

11. Dispositif selon l'une des revendications précédentes, **caractérisé en ce que** l'extrémité distale de la tige dé cathéter ne peut faire saillie de l'élargissement de ballonnet distal ou intrarectal entourant l'extrémité de tige de manière potentiellement traumatisante pour l'intestin, **en ce que** la pointe distale de la tige de cathéter en sa position de repos non déviée se trouve complètement à proximité d'un plan (Z) traversé perpendiculairement par l'axe de symétrie (X), ledit plan étant distalement complètement tangent à la partie de ballonnet intrarectale.

12. Dispositif selon la revendication 1 ou 2, **caractérisé en ce que** pour une autre déviation axiale, dirigée vers l'intestin de l'extrémité de tige proximale de la tige portant le ballonnet passe à l'état d'une compression axiale simple ou multiple, en particulier lors lors de déplacements de plus longues distances, dirigés vers l'intestin de la tige, ou lors d'une dislocation complète de toute la partie portant le ballonnet du drainage à partir du placement transanal dans l'intestin, ou lors d'une collision forcée des parties de drainage avant avec des parties de la paroi intestinale.

**13.** Dispositif selon l'une des revendications précédentes, **caractérisé par** une plage de tolérance avec un jeu relatif simple entre la tige de cathéter et le ballonnet, c'est-à-dire à une force de rappel minimale $F_r \approx 0$, pour une déviation x par rapport à l'état neutre, sans exercice de force d'une valeur jusqu'à une première valeur limite $G_1$:

$$x \leq G_1 \Rightarrow F_r = 0 \pm \varepsilon,$$

soit $|\varepsilon| \leq 10$ g, de préférence $|\varepsilon| \leq 5$ g, en particulier $|\varepsilon| \leq 2$ g.

**14.** Dispositif selon l'une des revendications précédentes, **caractérisé par** une force de rappel due à une déformation de la partie de ballonnet à l'intérieur du rectum, c'est-à-dire à une force de rappel $F_r$ de l'enveloppe de ballonnet, pour une déviation x par rapport à l'état neutre, sans exercice de force, d'une valeur entre une première valeur limite $G_1$ et une seconde valeur limite $G_2$ :

$$G_1 \leq x \leq G_2 \Rightarrow F_r \approx C_H * (x - G_1),$$

soit $C_H$ correspondant à la constante de rappel de l'enveloppe de ballonnet gonflée sans pression.

**15.** Dispositif selon l'une des revendications précédentes, **caractérisé par** une force de rappel due à une déformation (par exemple, flambage axial ou compression) de la tige de cathéter, c'est-à-dire à une force de rappel $F_r$ de la tige de cathéter, pour une déviation, x par rapport à l'état neutre, sans exercice de force, d'une valeur entre au-dessus de la seconde valeur limite $G_2$ :

$$G_2 \leq x \Rightarrow F_r = C_K * (x - G_2),$$

soit correspondant à la (plus petite) constante de rappel de la tige de cathéter.

Fig.1

Fig.2

## Fig.3a

## Fig.3b

## Fig.3c

Fig.4

Fig.5

Fig.6

Fig.7

## Fig.8

## Fig.9

## Fig.10

Fig.11

Fig.12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008103788 A
- DE 102004033425 B4
- WO 2007118621 A1